# EUROPÄISCHE PATENTSCHRIFT

(11) **EP 0 594 100 B1**
(45) Veröffentlichungstag und Bekanntmachung des Hinweises auf die Patenterteilung: **17.07.1996**
(21) Anmeldenummer: 93116781.1
(22) Anmeldetag: 18.10.1993
(51) Int. Cl.: C07C 209/60, C07C 211/23

(54) **Verfahren zur Herstellung von Hexa-2,4-diin-1,6-diaminen**
Process for the preparation of hexa-2,4-diin-1,6-diamines
Procédé pour la préparation des hexa-2,4-diin-1,6-diamines

(30) Priorität: 23.10.1992 DE 4235886
(43) Veröffentlichungstag der Anmeldung: 27.04.1994
(73) Patentinhaber: BASF Aktiengesellschaft, D-67063 Ludwigshafen (DE)
(72) Erfinder: Rittinger, Stefan, Dr., D-6700 Ludwigshafen (DE); Rieber, Norbert, Dr., D-6800 Mannheim 51 (DE)

(56) Entgegenhaltungen:
- DE-C- 879 990
- JOURNAL OF MEDICINAL CHEMISTRY Bd. 17, Nr. 3 , 1974 , WASHINGTON US Seiten 355 - 358 J.G. CANNON ET AL '1,6-DIAMMONIUM-2,4-HEXADIYNE ANALOGS OF HEXAMETHONIUM'

## Beschreibung

Die vorliegende Erfindung betrifft ein Verfahren zur Herstellung von Hexa-2,4-diin-1,6-diaminen durch Umsetzung von Diacetylen mit Carbonylverbindungen und sekundären Aminen bei einem pH-Wert von 1 bis 7 in Gegenwart eines polaren Lösungsmittels und katalytische Mengen eines Cu⁺-Salzes.

Aus der DE-A-879 990 ist ein Verfahren zur Herstellung von Hexa-2,4-diin-1,6-diaminen durch Umsetzung von Diacetylen mit Aldehyden oder Ketonen und sekundären Aminen bekannt, bei dem die Ausbeuten zu wünschen übrig lassen. Ohne Katalysatorzusatz oder bei Zusatz von Silberverbindungen bilden sich nur geringe Mengen der gewünschten Produkte.

Die in der DE-A-877 453 beschriebene Vorgehensweise zur Verbesserung des Diacetylenumsatzes (Kondensation und Rückführung nicht umgesetzten und ausgegasten Diacetylens) ist aus Sicherheitsgründen im technischen Maßstab nicht praktikabel.

Der vorliegenden Erfindung lag daher die Aufgabe zugrunde, den zuvor genannten Nachteilen abzuhelfen.

Demgemäß wurde ein neues und verbessertes Verfahren zur Herstellung von Hexa-2,4-diin-1,6-diaminen der allgemeinen Formel I 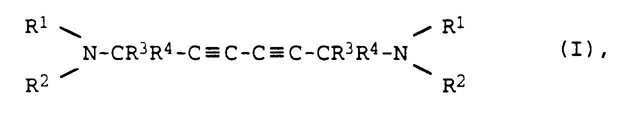
in der R¹, R², R³ und R⁴ C₁- bis C₂₀-Alkyl und R³ und R⁴ zusätzlich Wasserstoff bedeuten, durch Umsetzung von Diacetylen der Formel II

H-C≡C-C≡C-H (II),

mit Carbonylverbindungen der allgemeinen Formel III 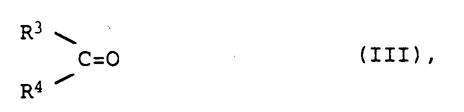
in der R³ und R⁴ die obengenannten Bedeutungen haben, und sekundären Aminen der allgemeinen Formel IV 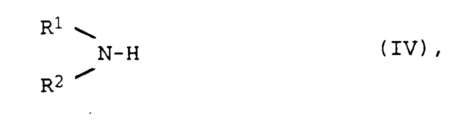
in der R¹ und R² die obengenannten Bedeutungen haben, in Gegenwart von Cu⁺-Katalysatoren, gefunden, welches dadurch gekennnzeichnet ist, daß man die Umsetzung bei einem pH-Wert von 1 bis 7 in Gegenwart eines polaren Lösungsmittels bei Temperaturen von -50° bis 150°C und Drücken von 0,01 bis 1,4 bar durchführt.

Das erfindungsgemäße Verfahren kann wie folgt durchgeführt werden:
Die Carbonylverbindungen III gegebenenfalls in Wasser oder wäßriger Lösung kann mit einem polaren Lösungsmittel in Gegenwart von 0,001 bis 5 Gew.-%, bevorzugt 0,005 bis 2 Gew.-%, besonders bevorzugt 0,01 bis 1 Gew.-% Cu⁺-Salzen vorgelegt und die sekundären Aminen IV unter Temperaturkontrolle von vorteilhaft nicht mehr als 120°C, bevorzugt 20 bis 100°C, besonders bevorzugt 40 bis 80°C zugetropft werden. In die entstandene Reaktionsmischung kann bevorzugt mit Säuren oder Basen auf einen pH-Wert von 1 bis 7, bevorzugt 2 bis 6, besonders bevorzugt 3 bis 5 gebracht werden und das Diacetylen bei Temperaturen von -50° bis 150°C, bevorzugt -40° bis 130°C, besonders bevorzugt 30° bis 100°C und Drücken von 0,01 bis 1,4 bar, bevorzugt 0,1 bis 1,2 bar, besonders bevorzugt bei Atmosphärendruck (Normaldruck) darin eingegast werden.

Als Cu⁺-Salze (Katalysatoren) eignen sich in der Regel alle Cu⁺-Salze, insbesondere Cu⁺-Halogenide wie CuCl und CuBr.

Das vorliegende Verfahren eignet sich im besonderen zur Verwertung von diacetylenhaltigen Teilströmen, wie sie technisch üblicher Weise bei der Zerlegung des Spaltgases aus der Spaltung von Kohlenwasserstoffen unter den Bedingungen der Acetylensynthese anfallen (Ullmann's Encycl. of Indust. Chem., V. Ed., A1, 1985). Die dabei teils gasförmig, teils flüssig anfallenden Gemische höherer Acetylene lassen sich im erfindungsgemäßen Verfahren ebenfalls verwenden.

Das Gasgemisch aus der Spaltgasaufbereitung hat gewöhnlich gegenüber der Umgebung leicht erhöhte Betriebstemperatur. Die Vorabscheidung von leicht kondensierbaren Gasbestandteilen in Abscheidebehältern in der Reaktorzuleitung bei Umgebungstemperatur verbessert die Reinheit des Rohproduktes.

Die Umsetzung kann sowohl diskontinuierlich als auch bevorzugt kontinuierlich in der Gasphsase oder bevorzugt in der Flüssigphase durchgeführt werden. Bei Nutzung eines gasförmigen Teilstroms sind Verfahren, die bekanntermaßen eine gute Gasverteilung in Flüssigkeiten erzielen, z.B. die Verwendung von Apparateteilen wie Begasungsring, Lochboden, verdichtende Begasungseinrichtungen, Sprühreaktor oder Absorberturm vorteilhaft anzuwenden. Als polare Lösungsmittel eignen sich Lactame, z.B. Pyrrolidone wie N-Methylpyrrolidon, Lactone, z.B. 5- bis 8-Ringlactone wie Butyrolacton, Ester, z.B. C₁- bis C₂₀-Carbonsäurealkylester wie Ameisensäuremethylester, Essigsäuremethylester, Propionsäuremethylester, Ameisensäureethylester, Essigsäureethylester und Propionsäureethylester, Säureamide, z.B. Dialkylformamide wie Dimethylformamid, Alkohole, z.B. C₁- bis C₂₀-Alkanole wie Methanol und Ethanol, alkylierte Harnstoffe oder Glykolether wie Ethylenglykoldiethylether.

Als Säuren oder Basen zur pH-Kontrolle eignen sich unter den Säuren beispielsweise Mineralsäuren wie Salzsäure, Schwefelsäure, Phosphorsäure, und unter den Basen beispielsweise Hydroxide wie Natriumhydroxid, Kaliumhydroxid, sowie Gemische aus diesen.

Die Substituenten R¹, R², R³ und R⁴ haben die folgenden Bedeutungen:
R¹, R², R³ und R⁴
- unabhängig voneinander
- C₁- bis C₂₀-Alkyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl,
R³ und R⁴
- zusätzlich Wasserstoff.

Tertiäre 1,6-Diamine sind interessante Zwischenprodukte für Anwendungen wie beispielsweise Weichmacher, Korrosionsinhibitoren, Textilhilfsmittel, Emulgatoren oder Urethanisierungskatalysatoren.

### Beispiele

Das Diacetylen im Gasstrom vor bzw. nach der Reaktion wurde gaschromatographisch auf einer gepackten Säule (20 % Reoplex® 400 auf Chromosorb® PAW) mit Trägergas N₂ (35 ml/min.) und FID-Detektion bestimmt. Die Konzentrationen sind in Vol-% angegeben. Die Diacetylenbestimmung in flüssiger Phase sowie die Ermittlung der Produktgehalte erfolgte gaschromatographisch auf einer Kapillarsäule HP1 mit einem WLD-Detektor.

### Beispiel 1

Zu 34 g (0,41 mol) Formalin (36%ige wäßrige Formaldehydlösung) wurden 32,6 g (0,4 mol) Dimethylaminhydrochlorid so zugegeben, daß die Innentemperatur 75°C nicht überstieg. Nach Zugabe von 50 ml N-Methylpyrrolidin wurde der pH-Wert der Lösung mit NaOH-Lösung gegen eine pH-Elektrode auf 4,4 eingestellt und 0,1 g CuCl zugesetzt. Anschließend wurde ein ca. 10 % Diacetylen enthaltender Propangasstrom bei 60°C eingegast. Eine GC-Analyse des Abgases ergab einen Diacetylengehalt von 6 % entsprechend ca. 40 %iger Abreicherung. Nach 4 h war in der Reaktionslösung kein Ausgangsprodukt mehr nachweisbar (GC). Der Reaktionsaustrag wurde abgekühlt und mit Natronlauge alkalisch gestellt. Man erhielt 140 g bestehend aus 15 Gew.-% (GC) (Ausbeute: 64 %) N,N,N'N'-tetramethyl-2,4-hexadiin-1,6-diamine und 3 Gew.-% (Ausbeute: 9.3 %) 1,1-Dimethylaminopenta-2,4-diin.

### Beispiel 2

In einer Versuchsreihe analog Beispiel 1 wurde der pH-Wert variiert. Die Einstellung des pH-Wertes nach der Methylolbildung (hergestellt aus Dimethylaminhydrochlorid und Formalin) wurde durch Zutropfen von wäßriger NaOH- bzw. HCl-Lösung gegen eine pH-Elektrode erreicht.

Die Ergebnisse sind in der nachfolgenden Tabelle zusammengestellt:
Abhängigkeit der Ausbeute an Diaminohexadiin vom pH-Wert

| pH-Wert | Ausbeute |
|---|---|
| 2 | 20 % |
| 7 | 20 % |
| 10 | 15 % |

### Beispiel 3

Zu 28,4 g Dimethylamino-hydrochlorid wurden 28,8 g Formalinlösung so zugegeben, daß die Innentemperatur 75°C nicht überstieg. Der pH-Wert der Lösung wurde mit NaOH-Lösung gegen eine pH-Elektrode auf 5,2 eingestellt und 0,17 g CuCl zugesetzt. Anschließend wurden 50 g einer 17.4 % Diacetylen enthaltenden N-Methylpyrrolidon-Waschlösung aus der Spaltgasaufbereitung einer Acetylenanlage binnen 15 min bei 60°C zugetropft. Nach 3 h war kein Diacetylen mehr nachweisbar (GC). Der Reaktionsaustrag wurde abgekühlt und mit Natronlauge alkalisch gestellt. Man erhielt 153 g bestehend aus 8,9 Gew.-% (Ausbeute: 48 %) Tetramethyldiaminohexadiin.

### Beispiel 4

In einer 2 l-Blasensäule mit Gaseinleitungsfritte und Rückflußkühlung wurden 1000 g N-Methylpyrrolidon vorgelegt. Hierzu wurde eine Lösung von Dimethylaminomethylol gegeben, die separat wie in Beispiel 1 beschrieben aus 42 g Formalin und 40,8 g Dimethylaminohydrochlorid hergestellt worden war. Nach Zugabe von 0,3 g CuCl wurde der pH-Wert auf 4 eingestellt und bei 60°C 5 l/h eines diacetylenhaltigen Gasstromes aus der Spaltgasaufbereitung einer Acetylenanlage eingeleitet, dessen durchschnittlicher Diacetylengehalt 12 % betrug. Nach ca. 8 h und einem Gesamtdurchsatz von 37,5 l Einsatzgas war kein Alkylol mehr nachweisbar (GC). Der Reaktionsaustrag wurde abgekühlt und mit Natronlauge alkalisch gestellt. Man erhielt 1102 g bestehend aus 29,5 g (72%) Tetramethyldiaminohexadiin. Dimethylaminopentadiin wurde nur in Spuren nachgewiesen.

### Beispiel 5

Zu 13 g Dimethylamino-hydrochlorid wurden 13,1 g Formalinlösung so zugegeben, daß die Innentemperatur 75°C nicht überstieg und der pH-Wert mit NaOH gegen eine pH-Blektrode auf 4 eingestellt. Anschließend wurden 20 g einer 20 % Diacetylen enthaltenden N-Methylpyrrolidin-Waschlösung aus der Spaltgasaufbereitung einer Acetylenanlage binnen 15 min zu der auf 60°C temperierten, katalysatorfreien Reaktionslösung zugetropft. Nach 30 h war kein Diacetylen mehr nachweisbar und die Reaktionslösung wurde gekühlt und mit NaOH alkalisch gestellt. Man erhielt 60,6 g bestehend aus 1,6 Gew.-% (Ausbeute: 7,4 %) Tetramethyldiaminohexadiin und 1,6 Gew.-% (Ausbeute: 5 %) Dimethylaminopentadiin.

## Patentansprüche

1. Verfahren zur Herstellung von Hexa-2,4-diin-1,6-diaminen der allgemeinen Formel I 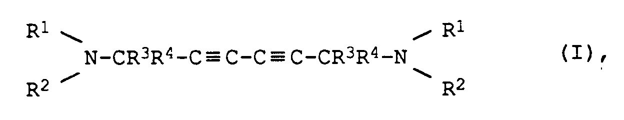 in der R¹, R², R³ und R⁴ C₁- bis C₂₀-Alkyl und R³ und R⁴ zusätzlich Wasserstoff bedeuten, durch Umsetzung von Diacetylen der Formel II
H-C≡C-C≡C-H (II),
mit Carbonylverbindungen der allgemeinen Formel III 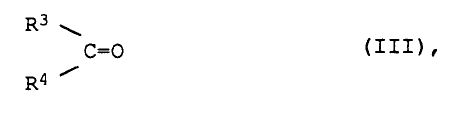 in der R³ und R⁴ die obengenannten Bedeutungen haben, und sekundären Aminen der allgemeinen Formel IV 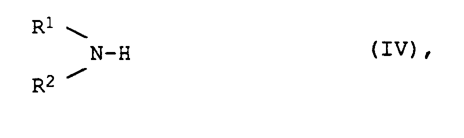 in der R¹ und R² die obengenannten Bedeutungen haben, in der Gegenwart von Cu⁺-Katalysatoren, dadurch gekennnzeichnet, daß man die Umsetzung bei einem pH-Wert von 1 bis 7 in Gegenwart eines polaren Lösungsmittels bei Temperaturen von -50° bis 150°C und Drücken von 0,01 bis 1,4 bar durchführt.

2. Verfahren zur Herstellung von Hexa-2,4-diin-1,6-diaminen I nach Anspruch 1, dadurch gekennzeichnet, daß man die Umsetzung bei einem pH-Wert von 2 bis 6 durchführt.

3. Verfahren zur Herstellung von Hexa-2,4-diin-1,6-diaminen I nach Anspruch 1, dadurch gekennzeichnet, daß man die Umsetzung bei einem pH-Wert von 3 bis 5 durchführt.

4. Verfahren zur Herstellung von Hexa-2,4-diin-1,6-diaminen I nach Anspruch 1, dadurch gekennzeichnet, daß man die Umsetzung bei bei Temperaturen von -40° bis 130°C durchführt.

5. Verfahren zur Herstellung von Hexa-2,4-diin-1,6-diaminen I nach Anspruch 1, dadurch gekennzeichnet, daß man die Umsetzung bei Temperaturen von 30° bis 100°C durchführt.

6. Verfahren zur Herstellung von Hexa-2,4-diin-1,6-diaminen I nach Anspruch 1, dadurch gekennzeichnet, daß man die Umsetzung bei Drücken von 0,1 bis 1,2 bar durchführt.

7. Verfahren zur Herstellung von Hexa-2,4-diin-1,6-diaminen I nach Anspruch 1, dadurch gekennzeichnet, daß man die Umsetzung bei Atmosphärendruck durchführt.

8. Verfahren zur Herstellung von Hexa-2,4-diin-1,6-diaminen I nach Anspruch 1, dadurch gekennzeichnet, daß man als polare Lösungsmittel Lactame, Lactone, Ester, Säureamide, Alkohole, alkylierte Harnstoffe und/oder Glykolether einsetzt.

## Claims

1. A process for preparing 2,4-hexadiyne-1,6-diamines of the formula I 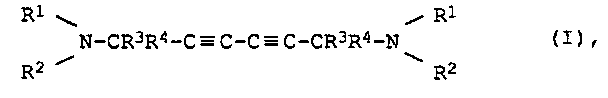 where R¹, R², R³ and R⁴ are each C₁-C₂₀-alkyl and R³ and R⁴ are each additionally hydrogen, by reacting diacetylene of the formula II
H-C≡C-C≡C-H (II),
with carbonyl compounds of the formula III 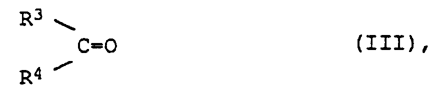 in which R³ and R⁴ have the abovementioned meanings, and secondary amines of the formula IV 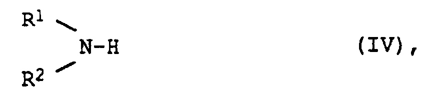 where R¹ and R² have the abovementioned meanings, in the presence of Cu⁺ catalysts, which comprises carrying out the reaction at a pH of from 1 to 7 in the presence of a polar solvent at from -50° to 150°C and under from 0.01 to 1.4 bar.

2. A process for preparing 2,4-hexadiyne-1,6-diamines I as claimed in claim 1, wherein the reaction is carried out at a pH of from 2 to 6.

3. A process for preparing 2,4-hexadiyne-1,6-diamines I as claimed in claim 1, wherein the reaction is carried out at a pH of from 3 to 5.

4. A process for preparing 2,4-hexadiyne-1,6-diamines I as claimed in claim 1, wherein the reaction is carried out at from -40° to 130°C.

5. A process for preparing 2,4-hexadiyne-1,6-diamines I as claimed in claim 1, wherein the reaction is carried out at from 30° to 100°C.

6. A process for preparing 2,4-hexadiyne-1,6-diamines I as claimed in claim 1, wherein the reaction is carried out under from 0.1 to 1.2 bar.

7. A process for preparing 2,4-hexadiyne-1,6-diamines I as claimed in claim 1, wherein the reaction is carried out under atmospheric pressure.

8. A process for preparing 2,4-hexadiyne-1,6-diamines I as claimed in claim 1, wherein lactams, lactones, esters, amides, alcohols, alkylated ureas and/or glycol ethers are employed as polar solvents.

## Revendications

1. Procédé de préparation d'hexa-2,4-diyne-1,6-diamines de la formule générale I 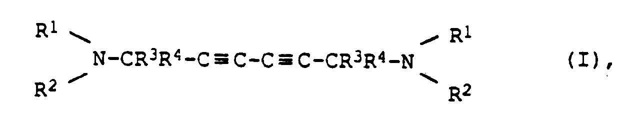 dans laquelle R¹, R², R³ et R⁴ représentent des radicaux alkyle en C₁ à C₂₀ et R³ et R⁴ représentent supplémentairement des atomes d'hydrogène, par la réaction du diacétylène de la formule II
H-C≡C-C≡C-H (II)
avec des composés carbonylés de la formule générale III 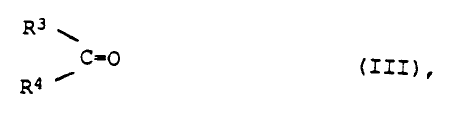 dans laquelle R³ et R⁴ possèdent les significations qui leur ont été attribuées ci-dessus et des amines secondaires de la formule générale IV 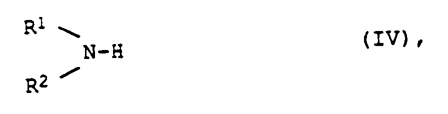 dans laquelle R¹ et R² possèdent les significations qui leur ont été attribuées ci-dessus, en présence de catalyseurs à Cu⁺, caractérisé en ce que l'on entreprend la réaction à une valeur de pH de 1 à 7, en présence d'un solvant polaire, à des températures de -50°C à 150°C et sous des pressions de 0,01 à 1,4 bar.

2. Procédé de préparation d'hexa-2,4-diyne-1,6-diamines I suivant la revendication 1, caractérisé en ce que l'on entreprend la réaction à une valeur de pH de 2 à 6.

3. Procédé de préparation d'hexa-2,4-diyne-1,6-diamines I suivant la revendication 1, caractérisé en ce que l'on entreprend la réaction à une valeur de pH de 3 à 5.

4. Procédé de préparation d'hexa-2,4-diyne-1,6-diamines I suivant la revendication 1, caractérisé en ce que l'on entreprend la réaction à des températures de -40°C à 130°C.

5. Procédé de réparation d'hexa-2,4-diyne-1,6-diamines I suivant la revendication 1, caractérisé en ce que l'on entreprend la réaction à des températures de 30°C à 100°C.

6. Procédé de préparation d'hexa-2,4-diyne-1,6-diamines I suivant la revendication 1, caractérisé en ce que l'on entreprend la réaction sous des pressions de 0,1 à 1,2 bar.

7. Procédé de préparation d'hexa-2,4-diyne-1,6-diamines I suivant la revendication 1, caractérisé en ce que l'on entreprend la réaction à la pression atmosphérique.

8. Procédé de préparation d'hexa-2,4-diyne-1,6-diamines I suivant la revendication 1, caractérisé en ce que l'on utilise, à titre de solvants polaires, des lactames, des lactones, des esters, des amides d'acides, des alcools, des urées alkylées et/ou des éthers glycoliques.
